## Europäisches Patentamt

## European Patent Office

## Office européen des brevets

(19)

(11) Veröffentlichungsnummer: **0 069 203**
**B1**

(12)

# EUROPÄISCHE PATENTSCHRIFT

(45) Veröffentlichungstag der Patentschrift:
13.08.86

(51) Int. Cl.⁴: **C 07 D 303/16,** C 07 D 301/00

(21) Anmeldenummer: **82103368.5**

(22) Anmeldetag: **21.04.82**

(54) **Verfahren zur Herstellung von aromatischen Glycidylestern.**

(30) Priorität: **04.07.81 DE 3126411**

(43) Veröffentlichungstag der Anmeldung:
**12.01.83 Patentblatt 83/2**

(45) Bekanntmachung des Hinweises auf die Patenterteilung:
**13.08.86 Patentblatt 86/33**

(84) Benannte Vertragsstaaten:
**CH DE FR GB LI NL**

(56) Entgegenhaltungen:
**US-A-3 454 531**
**US-A-3 475 382**
**US-A-4 228 084**

**CHEMICAL ABSTRACTS, Band 94, Nr. 17, 27. April 1981, Seite 744, Nr. 139598k, Columbus, Ohio, USA Helv. Chim. Acta 60 (1977), S. 1845-1860**

**Die Akte enthält technische Angaben, die nach dem Eingang der Anmeldung eingereicht wurden und die nicht in dieser Patentschrift enthalten sind.**

(73) Patentinhaber: **Degussa Aktiengesellschaft, Weissfrauenstrasse 9, D-6000 Frankfurt am Main 1 (DE)**

(72) Erfinder: **Nees, Friedbert, Dr. Dipl.- Chem., Fichtestrasse 4, D-7513 Stutensee 4 (DE)**
Erfinder: **Werle, Peter, Dr. Dipl.- Chem., Im Börner 43, D-6460 Gelnhausen 2 (DE)**
Erfinder: **Reissmann, Günther, Haselmühlweg 42, D-8750 Aschaffenburg (DE)**
Erfinder: **Merk, Wolfgang, Dr. Dipl.- Chem., Grünaustrasse 19, D-6450 Hanau 9 (DE)**

EP 0 069 203 B1

## Beschreibung

Glycidylester aromatischer Carbonsäuren können in vielfältiger Weise in der Klebstoff, Kunstharz- und Lackindustrie eingesetzt werden und werden z.B. in diversen Patentschriften für die Herstellung von hitzehärtbaren Überzugsmassen, z.B. Pulverlacken als Vernetzungskomponente beansprucht.

Grundsätzlich sind mehrere Methoden zur Herstellung dieser Verbindungen bekannt. Wie in der Angewandten Makromolekularen Chemie 31, 83 (1973) angeführt, lassen sich Glycidylester herstellen durch

1. Reaktion von Epichlorhydrin (ECH) mit Carbonsäuren und nachfolgender Dehyrdrohalogenierung Jap. Anm. SHO-45-39.393, US-A-3.576.827, DE-B-2.126.280, DE-B-1.643.777

2. Reaktion von Epichlorhydrin mit Salzen von Carbonsäuren, Ang.Makrom.Chemie 31 (1973) s. 83-113 (Nr. 443)

3. durch Epoxidation von Allylestern, EP-A-8 112

4. durch Reaktion von Säurechloriden mit 2,3-Epoxypropanol (Glycid), J.Chem.Eng.Data Bd 11 Nr. 3, Zeile 66, S.447-448 (Sandler; By)

Weiterhin sind noch folgende in der Literatur beschriebene Möglichkeiten zu nennen:

5. Umsetzung eines Säurehydrids mit Epichlorhydrin

6. Umesterung von Säureestern mit Glycidylestern DE-A-2.107.084

7. Umesterung von Säureestern mit Glycid SU-A-405.880, DE-A-2.602.157

Alle bislang beschriebenen Methoden eignen sich entweder nicht für die großtechnische Darstellung oder sind, was die Qualität der Endprodukte betrifft, mit verschiedenen Nachteilen behaftet.

Die Dehydrohalogenierung der 2,3-Hydroxychlorpropylester, die durch die Umsetzung der Carbonsäure mit Epichlorhydrin erhalten werden, mit einem alkalischen Reagens (z.B. DE-B-1.030.824 und DE-B-3.576.827) oder durch Umepoxidation mit weiterem Epichlorhydrin (DE-B-1.643.777 und DE-B-2.126.280) führen im ersten Fall bei milden Reaktionsbedingungen zu keiner vollständigen Cl-Abspaltung, bei schärferen Bedingungen zu einer Ablösung der Esterbindung, so daß sich die gebildeten Glycidylester wieder abspalten (vgl. DE-B-1.030.824). Im zweiten Fall ist es, wie generell bei der Umsetzung mit Epichlorhydrin, nicht möglich, zu chloridfreien Endprodukten zu gelangen, das Verfahren produziert ebenso wie die Umsetzung der Carbonsäureanhydride mit Epichlorhvdrin (Jap.Anm. -SHO-45-39393) große Mengen an organisch belastetem Salz.

Außerdem sind alle genannten Verfahren aufwendig in ihrem technischen Ablauf, weil die Reaktion der Alkalisalze mit Epichlorhydrin (z.B. DE-C-1.081.013 und US-A-2.448.602) keine wesentlichen Vorteile bringt, zumal ein weiterer Verfahrensschritt, nämlich die Herstellung des betreffenden Carbonsäuresalzes notwendig ist. Der Gehalt an Rest-Chlor liegt bei allen Prozessen recht hoch und kann mehrere Prozent betragen. Die Epoxydation der Allylester mit perverbindungen wird in mehreren Patentschriften erwähnt (vgl. z.B. GB-A-862.588, DE-B-1.082.263, S.R. gandler und F.R.Berg, J.Chem. und Eng.Data, Vol. 11 S.447 (1966) EP-A-8.112).

Diese Methoden werden bislang nur beschränkt angewandt, da die Verfahren aufwendig und technologisch schwierig sind. Die Umsetzung von Säurechloriden mit Glycid bringt wegen der schlechten Verfügbarkeit der Chloride und des hohen Anfalls an Alkali- oder Aminsalzen keine Vorteile hinsichtlich Qualität und Einfachheit des Reaktionsablaufs (vgl. z.B. F.Zetche und F.Aeschliman Helv.Chim.Acta 9, 708 (1929), US-A-3.073.804).

Die Umesterung von Säureestern mit niedermolekularen aliphatischen Glycidylestern.(DE-A-2.654.306 und DE-A-2.107.084) ist zwar möglich, doch scheitert das Verfahren für eine großtechnische Darstellung an der schlechten Verfügbarkeit der aliphatischen Glycidylester.

Glycidylester lassen sich auch durch Umsetzung von Glycid mit Estern von aromatischen Carbonsäuren, vorzugsweise von Methylestern, herstellen.

Dieses Verfahren ist technisch elegant und wenig aufwendig hinsichtlich der apparativen Ausstattung und Aufarbeitung der Mutterlaugen. Außerdem sind die notwendigen Ausgangskomponenten in großtechnischem Maßstab verfügbar.

Bislang nur unbefriedigend gelöst war die Frage der verwendbaren Katalysatoren. In der SU-A-405.880 wird Zinkacetat beansprucht. Die angegebenen Reaktionstemperaturen von 100 - 150°C führen jedoch zu einer raschen Polyaddition des Glycid. Beim Nacharbeiten der angegebenen Vorschrift wurden stets nur geringe Mengen an Diglycidylester erhalten, neben viel Ausgangsprodukt wirden erhebliche polymere Anteile festgestellt.

Zondler et al. beschreiben in Helv.Chim.Acta 60, 1845 (1977) sowie in der DE-A-2.602.157 die Umesterung von Carbonsäuremethylestern mit Glycid in Gegenwart verschiedener Thalliumsalze als Katalysatoren.

Ihre Untersuchungen an den Systemen $>>$gO, CdO, PbO, PbO$_2$, Sb$_2$O$_3$, Bi$_2$O$_3$, Ga$_2$O$_3$, In$_2$O$_3$, Mn(ac)$_2$, Hg(ac)$_2$, Pb(ac)$_2$, UO$_2$(ac)$_2$, TiO(acac)$_2$, In(acac)$_3$, Th(acac)$_4$, Ga(O-n-C$_4$H$_9$)$_4$, Ti(O-n-C$_4$H$_9$)$_4$ Ti(O-iso-C$_3$H$_7$)$_4$, (n-C$_4$H$_9$)Sn(ac)$_2$, (n-C$_4$R$_9$)$_3$Si-ac, n-C$_4$H$_9$-Sn-OOH und KCN zeigten für Dimethylterephthalat, daß alle Katalysatoren unterhalb 100 °C keine oder eine zu geringe Aktivität aufweisen, es ergaben sich daher nur niedrige Umsetzungsgrade.

Die in der obigen deutschen Offenlegungsschrift beanspruchten thalliumhaltigen Katalysatoren wie TINO$_3$, Tl$_2$O$_3$,

TIOCOCH$_3$ u.a. zeigen zwar eine gute Aktivität, jedoch ist es praktisch nicht möglich, den entstehenden Glycidylester in thalliumfreier Form zu isolieren. Aufgrund der bekanten Toxizität dieses Metalls und seiner Derivate ist der Einsatz solch thalliumhaltiger Ester stark eingeschränkt.

Der Erfindung lag somit die Aufgabe zugrunde, für das technisch einfache Verfahren der Umesterung von Methylestern mit Glycid Katalysatoren aufzufinden, die es erlauben aufgrund ihrer Aktivität und niedrigen Troxizität Glycidylester in großtechnischem Maße herzustellen.

Gerade die Umesterung mehrwersiger Carbonsäureester ist problematisch, da es gilt, die intermediär auftretenden Zwischenstufen, d.h. die Mischester, heiter vollständig in Richtung des reinen Glvcidylester umzusetzen. Die für die Reaktion von Monoestern angewandtern Katalysatoren sind nicht ohne weiteres für Polyester geeignet. Es hat sich vielmehr gezeigt, daß die für aromatische Carbonmonoester beanspruchten Katalysatoren wie NaOCH$_3$ (DT-A-2.107.084) andere basische Substanzen wie Hydroxide oder Cyanide, Ionenaustauscher, Säuren wie H$_2$SO$_4$, p-Toluolsulfonsäure nicht geeignet sind, reine Polyglycidylester zu erzeugen.

Die Umesterung mit Glycid führt vielmals nur zur Bildung der Monoglycidylester, z.B. erhält man beim Umestern von Dimethyltherephthalat mit 2 Mol Glycid in Gegenwart von KCN hauptsächlich den Monoester, neben Ausgangsprodukt und viel polymerem Material.

Eine Vielzahl von Alkalisalzen, die einer Überprüfung hinsichtlich ihrer katalytischen Aktivität unterzogen wurden, erwiesen sich als nicht geeignet. Dazu gehören u.a, die Anionen folgender Säuren:

PO$_4^{3-}$, HCO$_3^-$, HPO$_4^{2-}$, NO$_3^-$, NO$^{2-}$, BO$_2^-$, H$_2$PO$_2^-$, BH$_3$CN$^-$, SO$_4^{2-}$, SO$_3^{2-}$, JO$_4^-$, S$_2$O$_8^{2-}$, S$_2$O$_5^{2-}$, S$_2$O$_4^{2-}$.

Die Aufgabe des erfindungsgemäßen Verfahrens ist es daher, aromatische Mono- und vor allem Polycarbonsäureglycidylester durch Umesterung von aromatischen Carbonsäurealkylestern mit Glycid in sehr guten Ausbeuten und großer Reinheit herzustellen, und zwar unter Verwendung umweltfreundlicher Katalysatoren.

Es wurde nun gefunden, daß sich aromatische Mono- und Polycarbonsäureglycidylester durch Umsetzen der entsprechenden Carbonsäuremethyl- oder -äthylester mit Glycidol in Gegenwart von Katalysatoren herstellen lassen, wenn man die Umsetzung bei 50 - 80 °C und in Anwesenheit von 0,0001 - 0,01 Mol Katalysator pro Mol Carbonsäureester einsetzt und als Katalysatoren Alkalisalze der Stickstoffwasserstoffsäure, des Dicyanimids, der Cyansäure und/oder der Selenocyansäure verwendet.

Als Alkalimetalle kommen vor allem Li, Na und K infrage, in manchen Fällen auch NH$_4$.

Bevorzugt sind die Natrium- und Kaliumsalze.

Besonders bevorzugt sind Natriumacid, Kaliumcyanat, Natriumdicyanimid und des Valiumsalz der Selenoxyansäure.

Die Alkalisalze der genannten Pseudohalogenwasserstoffsäuren sind bei Raumtemperatur stabil.

Die erfindungsgemäß zu verwendenden Katalysatoren werden bevorzugt in Mengen von 0,002 - 0,004 Mol pro Mol Carbonsäurealkylester eingesetzt.

Die Herstellung von Glycidylmethacrylat durch Umesterung von Methylmethacrylat mit Glycid in Gegenwart eines Alkalicyanids, -cyanats, -thiocyanats oder -fulminats ist bekannt, siehe US-A-4,228,084 und C.A. 94 (1981), Seite 744, 139598k.

Ein Vorteil des erfindungsgemäßen Verfahrens liegt in der Möglichkeit, die Umesterung bei verhältnismäßig tiefen Temperaturen durchzuführen, d.h. bei 50 - 80 °C. Bevorzugt ist eine Temperatur von 60 °C.

Wenn die aromatischen Carbonsäureester in Glycid vollständig löslich sind, kann die Umesterung ohne Lösungsmittel vorgenommen werden; i.a. wird man aber Lösungsmittel einsetzen.

Als Lösungsmittel können beispielsweise Benzol, Toluol, Xylol, aber auch deren chlorierte Abkömmlinge, verwendet werden, sowie alle Lösungsmittel, in denem sich die Komponenten der Umesterung lösen lassen und die diesen Komponenten gegenüber inert sind.

So kommen auch z.B. Dimethyläther, Dioxan und Dimethylfornamid infrage. Bevorzugte Lösungsmittel sind Toluol und Xylol.

Polymerisationsinbilitoren sind i.a. nicht nötig.

Von den aromatischen Mono- und Polycarbonsäureestern waren besonders geeignet: die Methyl- oder Äthylester von Benzoesäure, Benzoldi - tri- und tetracarbonsäuren, Toluylsäure, die dreiphthalsäuren 1,8-Naphthalindicarbonsäure.

Bevorzugt sind die Methyl- oder Äthylester von Benzosäure, Benzoltricarbonsäuren und unter ihnen besonders Trimesinsäure, Phthalsäuren und unter ihnen besonders Terephthalsäure.

Glycid wird in handelsüblicher Form verwendet.

Die Umesterung wird unter normalem Druck durchgeführt. Wenn erforderlich, können aber auch erhöhter oder erniedrigter Druck angewendet werden; auf jeden Fall sollen die entstehenden Alkohole, meistens Methanol aus dem Reaktionsgemisch entfernt werden. Bei Anwennдung von normalem Druck ist hierfür dann verminderter Druck erforderlich.

Die aromatischen Carbonsäureester und Glycid können in stöchiometrischen Mengen eingesetzt werden; i.a. wird aber ein geringer Überschuß an Glycid von 0,1-0,2 Mol Glycid pro Mol Carboxylgruppe zweckmäßig sein.

Die Reaktionszeit beträgt i.a. 5-7 Stunden. Das Reaktionsende wird dünnschichtchromatographisch nachgewiesen.

Die Isolierung der Produkte erfolgt durch Abdestillation des Lösungsmittels oder weitestgehende Einengung mit nachfolgender Kristallisation. Im allgemeinen ist eine weitere Kristallisation nicht notwendig, da die nach dem erfindungsgemäßen Verfahren hergestellten Glycidylester hohe Reinheit und hohen Epoxidgehalt aufweisen.

Der technische Fortschritt des erfindungsgemäßen Verfahrens liegt einmal in den sehr guten Ausbeuten, außerdem auch in der großen Reinheit der Umesterungsprodukte. Dies trifft vor allem auch für sogenannte Mischester mehrbasischer Carbonsäuren zu, deren zwei oder mehr Carborylgruppen nicht nur Glycidreste, sondern auch die Alkylreste der eingesetzten aromatischen Carbonsäuren enthalten.

Ferner sind die erfindungsgemäß verwendeten Umesterungskatalysatoren nur schwach toxisch.

Die nachrolgenden Beispiele sollen das erfindungsgemäße Verfahren näher erläutern:

## Apparatur:

Auf einem 250 ml Dreihalskolben mit Innenthermometer befindet sich eine ca. 20 cm lange Füllkörperkolonne, auf der sich ein mit 40° C beheizter Intensivkühler anschließt. Dieser ist mit einem Bogenstück mit Thermometer verbunden, an den sich ein absteigender Sclilangenkühler, der mit Wasser gekühlt wird, sowie eine Vakuumwechselvorlage z.B. nach Anschütz-Thiele, anschließt. An der Vakuumwechselvorlage befindet sich ein Kolben, in einem Eis-Bad.

Die Komponentan werden zusammen mit Xylol in den Tolben gefüllt und auf 60° C erwärmt. Durch Anlage eines Vakuums (etwa 60-70 mbar) läßt sich das abgespaltene Methanol verdampfen und aus dem Reaktionsraum in die Vorlage übertreiben. Das Ende der Reaktion läßt sich durch dünnschichtchromatographische Analyse mit Kieselgelplatten für Dünnschichtchromatographie in einem Laufmittelsystem Aceton/Hexan 1:3 am Verschwinden der Ausgangs- und Zwischenester nachweisen.

## Beispiel 1:

17 g (0,125 mol) Benzoesäuremethylester und 10,6 g (0,143 mol) Glycid in 100 ml Xylol herden mit 0,07 g (4,9.10$^{-4}$mol) KSeCN bei 60°C umgesetzt. Nach etwa 5 h ist die Reaktion beendet. Die Rohausbeute an farblosem Benzoesäureglycidylester beträgt 21,6 g (97,% d.Th., bezogen auf den Säuremethylester), Epoxidgehalt 96,2 %.

## Beispiel 2

24,25 g (0.125 mol) Dimethylterephthalat (DMT), 21,3 g (0,287 mol) Glycid, 100 ml Xylol sowie 0,03 g (4,9-l-0$^4$mol) Natriumazid werden 6-7 h bei 60° C analog Beispiel 1 behandelt.

Nach dem Abbdestillieren des Xylol hinterbleiben 46,2 g (97,7,% d.Th. auf DMT bezogen) an farblosem Diglycidylterephtalat mit einem Epoxidgehalt von 96

## Beispiel 3

Ansatz analog Beispiel 2.
Als Katalysator werden 0.03 g (4,9. 10$^{-4}$ mol) NaN(CN)$_2$ eingesetzt.
Ausbeute 94,8-% d Th., Epoxidgehalt 94,1 %

## Beispiel 4

Ansatz analog Beispiel 2, jedoch werden nur 0,015 g (2,5-10 mol) Natriumazid eingesetzt.
Ausbeute 96,3 % d.Th., Epoxidgehalt 95,3 %.

## Beispiel 5

31,5 g (0,125 mol) 1,3,5-Benzoetricarbonsäuretrimethylester, 32 g (0,43 mol) Glycid und 0,04 g (4,910$^{-4}$mol) Kaliumcyanat werden wie in Beispiel 2 beschrieben behandelt. Rohausbeute 46,2 g (97,7 % d.Th. bezogen auf den Säureester) Epoxidgehalt 96,7 %.

## Patentansprüche

1. Verfahren zur Herstellung von aromatischen Mono- und Polycarbonsäureglycidylestern durch Umsetzen der entsprechenden Carbonsäuremethyl- oder -äthylester mit Glycidol in Gegenwart von Katalysatoren, dadurch gekennzeichnet, daß man die Umsetzung bei 50 - 80 ° C und in Anwesenheit von 0,0001 - 0,01 Mol Katalysator pro Mol Carbonsäureester einsetzt und als Katalysatoren Alkalisalze der Stickstoffwasserstoffsäure, des Dicyanimids, der Cyansäure und/oder der Selenocyansäure verwendet.

2. Verfahren nach Anspruch 1, dadurch gekennzeichnet, daß man Natriumazid, Natriumdicyanimid, Kaliumcyanat oder das Kaliumsalz der Selenocyansäure einsetzt.

**Claims**

1. A process for the production of aromatic mono and polycarboxylic acid glycidyl esters by reacting the corresponding carboxylic acid methyl or ethyl esters with glycidol in the presence of catalysts, characterised in that the reaction is carried out at from 50 to 80-C and in the presence of from 0.0001 to 0.01 mol of catalyst per mol of carboxylic acid ester, alkali metal salts of hydrazoic acid, dicyanimide, cyanic acid and/or selenocyanic acid being used as catalyst.

2. A process according to claim 1, characterised in that sodium azide, sodium dicyanimide, potassium cyanate or the potassium salt of selenocyanic acid are used.

**Revendications**

1°) Procédé pour la préparation d'esters glycidyliques d'acides mono- et poly-carboxyliques aromatiques, par réaction de l'ester méthylique ou éthylique de l'acide carboxylique correspondant avec du glycidol, en présence de catalyseurs, caractérisé en ce qu'on met en route la réaction à 50 à 80°C et en présence de 0,0001 à 0,01 mole de catalyseur par mole d'ester d'acide carboxylique, et que l'on utilise, comme catalyseurs, des sels alcalins d'acide hydroazoïque, du dicyanimide, de l'acide cyanique et/ou de l'acide sélénocyanique.

2°) Procédé suivant la revendication 1, caractérisé en ce que l'on utilise l'azidede sodium, le dicyanimide de sodium, le cyanate de potassium ou le sel de potassium de l'acide sélénocyanique.